Europäisches Patentamt

European Patent Office

Office européen des brevets

(11) Veröffentlichungsnummer: **0 264 695**

**A2**

(12) **EUROPÄISCHE PATENTANMELDUNG**

(21) Anmeldenummer: 87114448.1

(22) Anmeldetag: 03.10.87

(51) Int. Cl.⁴: **A61M 1/18** , A61M 1/34

(30) Priorität: **11.10.86 DE 3634763**

(43) Veröffentlichungstag der Anmeldung:
**27.04.88 Patentblatt 88/17**

(84) Benannte Vertragsstaaten:
**AT BE CH DE ES FR GB GR IT LI LU NL SE**

(71) Anmelder: **Magasi, Josef**
**Wendelinusstrasse 8**
**D-6902 Sandhausen(DE)**

(72) Erfinder: **Magasi, Josef**
**Wendelinusstrasse 8**
**D-6902 Sandhausen(DE)**

(74) Vertreter: **Meyer-Roedern, Giso, Dr.**
**Blumenstrasse 1**
**D-6900 Heidelberg(DE)**

(54) **Verfahren und Vorrichtung zur Blutreinigung.**

(57) Es wird eine Filtration des dem Patienten abgenommenen Bluts mit einem für Mittelmoleküle durchlässigen ersten Membransystem durchgeführt. Das gewonnene Filtrat wird einer zweiten Filtration mit einem nur für kleine Moleküle durchlässigen zweiten Membransystem unterworfen. Das Retanat wird abgeführt. Das vom ersten Membransystem überstellte gefilterte Blut wird dialysiert, und das dialysierte Blut mit dem Permeat des zweiten Membransystems vereinigt und ohne Zugabe von Substituat zum Patienten zurückgeführt. Eine hierzu geeignete Vorrichtung hat ein Gehäuse (10) mit drei Hohlfasermembrankammern (14, 16, 18). Die Strömungsverbindung zwischen den Kammern (14, 16, 18) wird durch abgedichtete Zonen (50, 52, 54, 64, 68) an das Gehäuse (10) beidendig verschließenden Deckeln (20, 22) hergestellt.

Fig. 1

EP 0 264 695 A2

## Verfahren und Vorrichtung zur Blutreinigung

Die Erfindung betrifft ein Verfahren und eine Vorrichtung zur Blutreinigung.

Das üblichste Verfahren zur Blutreinigung ist die Hämodialyse. Verschiedene Bauformen von Dialysatoren sind aus der Praxis bekannt. Mit der Dialyse erreicht man hohe Eliminationsraten von kleinen Molekülen, während die Eliminationsrate bei mittleren und großen Molekülen hinter den Zielvorstellungen zurückbleibt.

Ein anderes bekanntes Verfahren zur Blutreinigung ist die Hämofiltration. Hier ist die Eliminationsrate von Mittelmolekülen wünschenswert hoch, von kleinen Molekülen aber gering. Dem Patienten werden bei der Behandlung große Volumina Filtrat entzogen, das in der richtigen Menge durch sterile, nichtpyrogene und vorzugsweise an den Patienten individuell angepaßte Substitutionslösung ersetzt werden muß. Der damit einhergehende Aufwand ist hoch.

Es ist auch bereits ein als Hämodiafiltration bezeichnetes Kombinationsverfahren bekannt, bei dem man eine Hämodialyse und Hämofiltration gleichzeitig durchführt. Dieses Verfahren verbindet die Vorteile des konvektiven Massentransports bei der Hämofiltration mit denen des dialytischen Diffusionsprozesses, und man erreicht eine höhere Eliminationsrate von kleinen und Mittelmolekülen pro Zeit-und Volumeneinheit, als dies mit dem jeweiligen Einzelverfahren allein zu erreichen wäre. Niedermolekulare Substanzen werden bei der Hämodiafiltration vorwiegend durch Diffusion, und Mittelmoleküle durch Konvektion entfernt.

Auch bei den gegenwärtig mit der Hämodiafiltration unternommenen Versuchen werden aber in der Hämofiltrationsstufe vom Patienten erhebliche Mengen Filtrat gewonnen und zumeist im Postdilutionsverfahren durch eine Substitutionslösung ersetzt. Damit geht ein beträchtlicher Bedarf an Elektrolytlösung einher, und der apparative Aufwand und Überwachungsaufwand ist hoch.

Im Zusammenhang mit der Hämofiltration wurden bereits Überlegungen angestellt, das Filtrat mittels geeigneter Sorbentien on-line selektiv aufzuarbeiten und das gereinigte Filtrat dem Patienten wieder zu reinfundieren. Dieses Konzept ist aber komplex und aufwendig, da es auf den gegenwärtig zur Verfügung stehenden Verfahren der Sorbentienregeneration beruht, und es hat keinen Eingang in die Praxis gefunden.

Aufgabe der Erfindung ist es, ein Verfahren zur Blutreinigung und eine unaufwendige, mit konventionellen Fertigungstechniken zu erstellende und einfach zu bedienende Vorrichtung zu seiner Durchführung anzugeben, mit denen eine Blutreinigung ohne Zufuhr von Substitutionslösung möglich ist und die Funktion der natürlichen Niere in an die Physiologie des Patienten optimal angepaßter Weise nachgeahmt wird.

Diese Aufgabe wird gelöst durch ein Verfahren zur Blutreinigung, bei dem man eine Filtration des dem Patienten abgenommenen Bluts mit einem für Mittelmoleküle durchlässigen ersten Membransystem durchführt, das gewonnene Filtrat einer zweiten Filtration mit einem nur für kleinere Moleküle durchlässigen zweiten Membransystem unterwirft, das Retenat abführt, das vom ersten Membransystem überstellte gefilterte Blut dialysiert und das dialysierte Blut mit dem Permeat des zweiten Membransystems vereinigt und ohne Zugabe von Substituat zum Patienten zurückführt. Dieses Verfahren kann zumindest potentiell in gewerblichen Zentren nach Art einer "limited care"-Blutreinigung durchgeführt werden.

Weiter wird die Aufgabe gelöst durch eine Vorrichtung zur Blutreinigung mit einem ersten Membranmodul zur Filtration des dem Patienten abgenommenen Bluts unter Einsatz eines für Mittelmoleküle durchlässigen ersten Membransystems, mit einem zweiten Membranmodul zur Filtration des aus dem ersten Membranmodul kommenden Filtrats unter Einsatz eines für kleinere Moleküle durchlässigen zweiten Membransystems, wobei ein Auslaß für das Retinat vorhanden ist, und mit einem dritten Membranmodul zur Dialyse des vom ersten Membranmodul kommenden gefilterten Bluts, wobei ein Auslaß für das dialysierte Blut und ein Auslaß für das Permeat des zweiten Membranmoduls verbunden und an eine zum Patienten führende Reinfusionsleitung angeschlossen sind.

Die Erfindung hat alle Vorteile der Hämodiafiltration. Insbesondere wird eine hohe Eliminationsrate sowohl der klein-als auch der mittelmolekularen Substanzen erreicht, wobei sich die Elimination dieser beiden Molekülgruppen unabhängig voneinander steuern läßt. Die allgemeine Verträglichkeit ist besser, und die Behandlungszeit kürzer, als bei der herkömmlichen Dialyse. Bislang der Hämodiafiltration zugeschriebene Nachteile, insbesondere der hohe Bedarf an Substitutionsflüssigkeit und der mit ihrer Zuführung verbundene apparative Aufwand und Überwachungsaufwand werden vermieden. Die selektive Aufarbeitung und anschließende Reinfusion des bei der Filtration gewonnenen Filtrats in einer zweiten Filterstufe gibt dem Patienten effektiver Weise einen Großteil der nützlichen Substanzen des Filtrats wieder, während die Dialysestufe in dem noch notwendigen Umfang hinsichtlich des Elektrolythaushalts und der Flüssigkeitsbilanz kompensatorisch wirkt. Mit der Filtrations-Eingangsstufe ahmt die Erfindung die Aktion des natürlichen Glomerulus, und mit der Aufarbeitung und anschließenden Reinfusion des Filtrats eine tubuläre Aktion der Niere nach, so daß man im Ergebnis der Wunschvorstellung einer die Funktion einer natürlichen Niere nachahmenden künstlichen Niere nahekommt.

Das Membransystem des ersten Membranmoduls kann eine Durchlässigkeit für Moleküle mit einem Molekulargewicht bis größenordnungsmäßig ca. 50.000 Atommasseneinheiten, vorzugsweise bis ca. 30.000 Atommasseneinheiten haben. Die Membransysteme des zweiten und dritten Membranmoduls haben hingegen vorzugsweise eine Durchlässigkeit für Moleküle bis größenordnungsmäßig ca. 1800 Atommasseneinheiten. Die wirksame Membranfläche des ersten Membransystems kann größenordnungsmäßig ca. 0,4 bis 0,5 m², die des zweiten Membransystems größenordnungsmäßig ca. 0,4 bis 0,6 m², und die des dritten Membransystems größenordnungsmäßig ca. 0,9 m² betragen.

Die erfindungsgemäße Vorrichtung weist vorzugsweise drei in einem gemeinsamen Gehäuse untergebrachte Hohlfasermembran module auf. Mit derartigen, je ein Bündel von Membrankapillaren enthaltenden Modulen knüpft man in vorteilhafter Weise an herkömmliche Fertigungstechniken an. Die Anordnung der Membranmodulen in einem gemeinsamen Gehäuse bringt einen geringen Materialaufwand, eine Vereinfachung der Herstellung und einen kompakten Aufbau mit sich.

Das Gehäuse kann prismatisch oder zylindrisch und an beiden Enden mit Deckeln versehen sein, die eine selektive Strömungsverbindung zwischen den Membranmodulen herstellen. Dieser Aufbau ermöglicht es, das Gehäuse insbesondere durch Einsatz von verschiedenen Membranen universell für verschiedene Verfahren der Blutreinigung zu verwenden, bei denen unterschiedliche oder auch gar keine Verbindungen zwischen den Membranmodulen bestehen. Die erforderlichen Verbindungen lassen sich auf einfache Weise durch Verwendung eines entsprechenden Dekkels herstellen. Im Ergebnis kann man so aus dem Gehäuse und einem Satz Deckel baukastenartig verschiedene Vorrichtungen zur Blutreinigung zusammenbauen.

Das Gehäuse weist vorzugsweise drei beidendig vergossene, je ein Bündel Hohlfasern enthaltende Kammern auf, wobei die Hohlfasern mit einem an der äußern Stirnseite offenen Ende in der Vergußmasse eingebettet sind. Mit diesem Verguß macht die Erfindung in vorteilhafter Weise von einer Technik der Dialysatorherstellung Gebrauch. An den Deckeln können in deren aufgesetztem Zustand gegeneinander abgedichtete Zonen abgeteilt sein, über die eine Strömungsverbindung mit dem Hohlfaserinnern der einzelnen Membranmodule besteht. Mit diesem Verbindungszonen erreicht man eine gleichmäßige Verteilung der zu filtrierenden bzw. dialysierenden Flüssigkeit auf die Kapillaren eines Membranmoduls. Außerdem lassen sich die Zonen in unaufwendiger Weise geometrisch so gestalten, daß die gewünschte Strömungsverbindung der Membranmodule untereinander hergestellt wird.

Die den einzelnen Membranmodulen zugeordneten Kammern können in dem Gehäuse nebeneinander liegen und durch eine gemeinsame Außenwand des Gehäuses und zwei mittlere Trennwände begrenzt sein. Dieser Aufbau ist materialunaufwendig und kompakt. Vorzugsweise sind die Kammern kreiszylindrisch. Der Fertigungsaufwand, und insbesondere die Werkzeugkosten sind dadurch gering.

An der Außenwand des Gehäuses können zwei an das Hohlfaseräußere der zu dem dritten Membranmodul gehörigen Kammer führende Anschlüsse für die Dialysatströmung vorgesehen. Die Lage der Dialysatanschlüsse entspricht damit der bei herkömmlichen Dialysatoren, was den Umgang mit der erfindungsgemäßen Vorrichtung erleichtert und der Gefahr von Fehlanschlüssen entgegenwirkt.

Das Gehäuse kann einstückig gefertigt, und insbesondere auf unaufwendige Weise im Spritzgußverfahren aus Kunststoff gefertigt sein. Das Gehäuse kann aber auch aus zwei Halbschalen bestehen, die miteinander verklebt oder verschweißt sein können. Dieser Aufbau ist unaufwendig und kostengünstig.

An einem ersten Deckel der erfindungsgemäßen Vorrichtung können drei je einem der Membranmodule zugeordnete Zonen vorgesehen sein, die mit je einem Anschluß insbesondere in Form eines Anschlußstutzens kommunizieren. Es führt dann vom Hohlfaseräußern der zu dem zweiten Membranmodul gehörigen Kammer ein Kanal an die dem dritten Membranmodul zugeordnete Zone dieses Deckels, der im Betrieb der erfindungsgemäßen Vorrichtung unten zu liegen kommt und im folgenden als unterer Deckel bezeichnet wird. Die Vereinigung des Permeats aus dem zweiten Membranmodul mit dem dialysierten Blut erfolgt somit am unteren Ende der erfindungsgemäßen Vorrichtung, wodurch einem Aufsteigeñ von Blut in das zweite Membranmodul abgesehen von den herrschenden Druckverhältnissen auch durch die Schwerkraft entgegengewirkt wird. Blutkörperchen sind vergleichsweise schwere Partikel.

An einem zweiten Deckel der erfindungsgemäßen Vorrichtung ist vorzugsweise eine dem ersten und dritten Membranmodul gemeinsam zugeordnete Verbindungszone und innerhalb dieser eine dem zweiten Membranmodul zugeordnete Zone vorgesehen. Vom Hohlfaseräußern der zu dem ersten Membranmodul gehörigen Kammer führt dann ein Kanal an die dem zweiten Membranmodul zugeordnete Zone dieses Deckels, der im Betrieb der Vorrichtung oben zu liegen kommt und im folgenden als oberer Deckel bezeichnet wird. Die Ausbildung der gemeinsamen Verbindungszone an diesem oberen Deckel ermöglicht es, das gefilterte Blut aus dem ersten Membranmodul mit großem Strömungsquerschnitt an das Dialyse-Kompartiment zu überstellen. Weiter wird die erforderliche Verbindung hergestellt, um das Filtrat des ersten Membranmoduls in das Kapillarinnere des zweiten Membranmoduls einzuleiten.

Die erwähnten Kanäle können mit Sammelleitungen verbunden sein, die sich vorzugsweise in Gehäuselängsrichtung erstrecken. Eine erste Sammelleitung nimmt somit das Filtrat (Permeat) des ersten Membranmodule, und eine zweite Sammelleitung des Permeat des zweiten Membranmoduls auf. Durch das Vorsehen dieser Sammelleitungen wird ein im wesentlichen den hydrostatischen Druckverhältnissen entsprechendes Durchströmen der erfindungsgemäßen Vorrichtung erreicht und ein Massentransport entgegen der Stromrichtung verhindert.

Die Sammelleitungen können im Bereich der Trennwände zwischen den Gehäusekammern einstückig mit dem Gehäuse ausgebildet sein. Hier empfiehlt sich insbesondere eine Bauform, bei der Halbseiten der Sammelleitungen an den Halbschalen des Gehäuses ausgeformt sind. Entsprechende Halbschalen lassen sich einfach aus Kunststoff fertigen.

Die Sammelleitungen können aber auch von einem in die jeweilige Kammer eingelegten, endseitig offenen Rohr oder Schlauch gebildet sein. Eine solche Rohr-oder Schlauchleitung wird in herstellungstechnisch unaufwendiger Weise mit in die Vergußmasse an den Enden der Kammern eingebettet.

Die an den Deckeln ausgebildeten Verbindungszonen können im Mündungsbereich der das Permeat führenden Känale Einbuchtungen oder Ausbuchtungen haben. Über diese wird mit geringem Aufwand die gewünschte Anschlußverbindung hergestellt. Abwandlungen sind durch Änderung der Deckelgeometrie leicht möglich. Am besten werden die Zonen durch in Nuten des Deckels einsitzende Dichtringe, insbesondere O-Ringe, abgeteilt. Diese Anordnung genügt bei einfachem Aufbau höchsten Anforderungen an Hygiene und Dichtigkeit.

Die Erfindung wird im folgenden anhand eines in den Zeichnungen dargestellten Ausführungsbeispiels näher erläutert. Es zeigen:

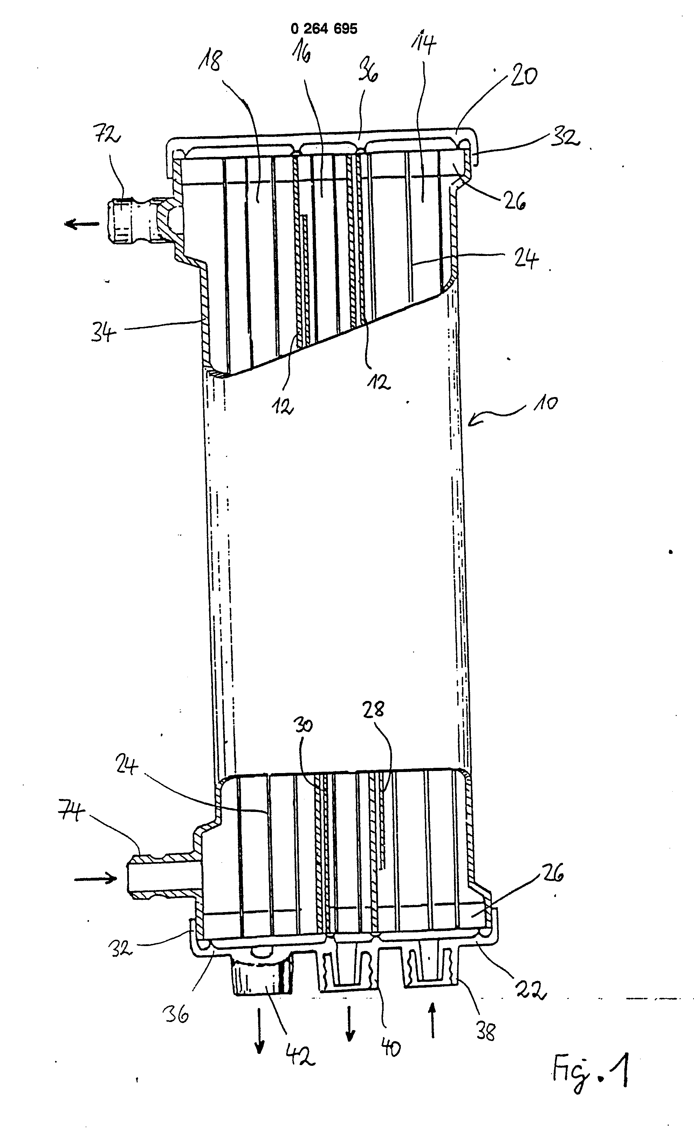

Fig. 1 die Vorderansicht einer teilweise aufgeschnittenen Vorrichtung zur Blutreinigung;

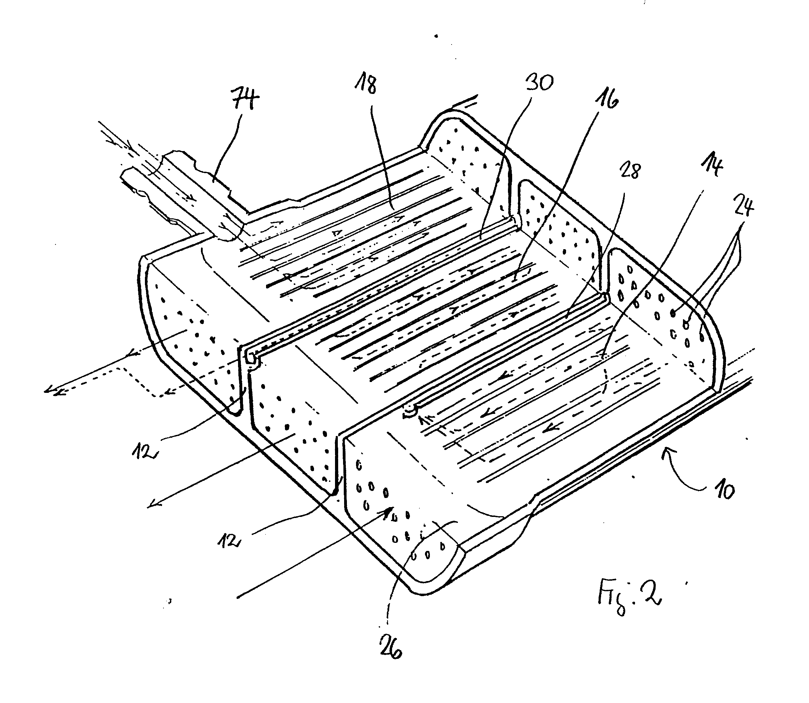

Fig. 2 einen perspektivischen Halbschnitt des unteren Endes der Vorrichtung mit den Strömungsverlauf verdeutlichenden Pfeilen;

Fig. 3 einen entsprechenden Halbschnitt des oberen Endes der Vorrichtung;

Fig. 4 die perspektivische Ansicht eines unteren Deckels der Vorrichtung; und

Fig. 5 die perspektivische Ansicht eines oberen Deckels der Vorrichtung.

Die in den Abbildungen gezeigte erfindungsgemäße Vorrichtung stellt den Membranblock einer Anlage zur Blutreinigung dar, zu der die bekannten Komponenten eines Dialysegeräts mit einem extrakorporalen Blutkreislauf und einem Dialysatkreis gehören. Entsprechende Membranblöcke für Dialysatoren sind handelsübliche, in aller Regel für eine einzige Dialysebehandlung zu verwendende Austauschteile. Soweit nicht nachstehend behandelte Besonderheiten vorliegen, kann man sich im Betrieb einen dieser bekannten Membranblöcke durch die erfindungsgemäße Vorrichtung ersetzt denken.

Die Vorrichtung hat ein zylindrisches Gehäuse 10 mit abgerundet rechteckigem Grundriß. In dem Gehäuse 10 sind durch zwei parallele, sich in Gehäuselängsrichtung erstreckende Trennwände 12 drei Kammern 14, 16, 18 abgeteilt. Die Kammern haben dadurch ebenfalls einen im wesentlichen recht eckigen Grundriß, können aber im Grundriß auch kreisrund sein. Stirnendig ist das Gehäuse 10 durch einen oberen Deckel 20 und einen unteren Deckel 22 verschlossen.

Die Kammern 14, 16, 18 enthalten je ein dicht gepacktes Bündel von Membrankapillaren 24, die sich in Gehäuselängsrichtung erstrecken. Die Kapillaren sind am oberen und unteren Ende des Gehäuses in eine Vergußmasse 26 eingebettet, durch die sie sich mit stirnseitig offenen Ende hindurch erstrecken. Die Vergußmasse 26 bildet einen abdichtenden Abschluß der Kammern 14, 16, 18, die einen dem Kapillaräußern zugeordneten Flüssigkeitsraum darstellen. Ein Flüssigkeitsanschluß zum Kapillarinnern wird an den Stirnseiten des Gehäuses 10 durch die Deckel 20, 22 hergestellt.

Die erste und zweite Kammer 14, 16 enthält je eine Sammelleitung 28, 30 für am Kapillaräußern befindliche Flüssigkeit. Die Sammelleitungen 28, 30 erstrecken sich in Längsrichtung des Gehäuses 10, und sie sind endseitig für den Eintritt von Flüssigkeit offen. Die Sammelleitung 28 der ersten Kammer 14 hat ein freies unteres Ende. Am oberen Ende ist sie durch die Vergußmasse 26 hindurchgeführt, aus der sie stirnseitig im Bereich des oberen Deckels 20 austritt. Umgekehrt hat die Sammelleitung 30 der zweiten Kammer 16 ein freies oberes Ende, und sie ist durch die Vergußmasse 26 am unteren Ende des Gehäuses 10 hindurchgeführt, so daß sie im Bereich des unteren Deckels 22 austritt. Die Sammelleitungen 28, 30 liegen jeweils im seitlichen Randbereich der entsprechenden Kammer 14, 16. Sie können auf unterschiedliche Weise realisiert sein, insbesondere durch in die Kammern 14, 16 eingelegte und zusammen mit den Kapillaren vergossene Rohre oder Schläuche.

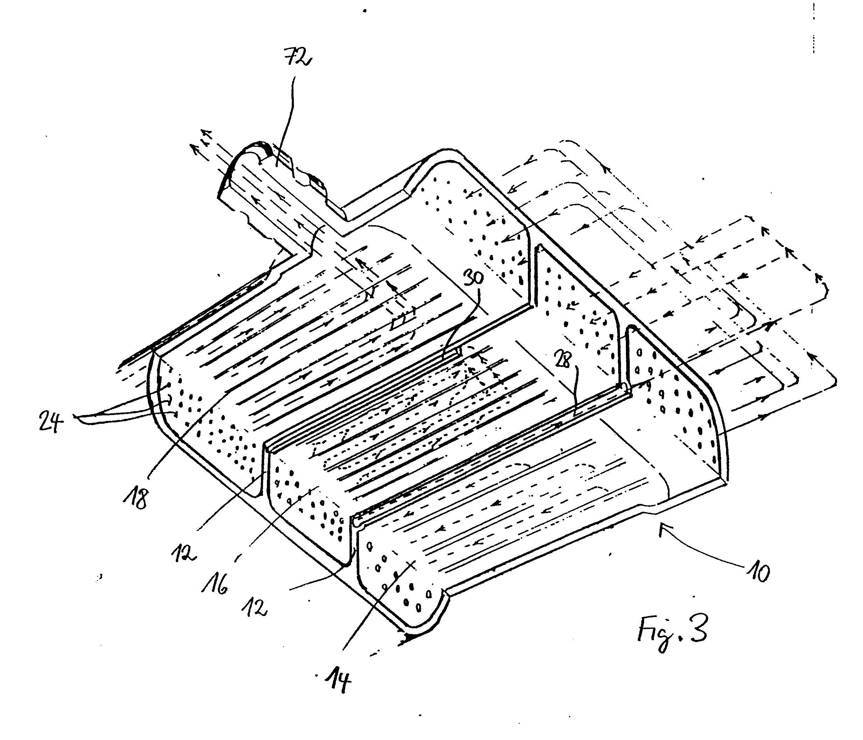

Wie in Fig. 3 dargestellt, ist es aber auch möglich, die Sammelleitungen 28, 30 in die Trennwände 12 des Gehäuses 10 einzuformen. Das Gehäuse 10 kann insbesondere aus zwei Halbschalen aufgebaut sein, die in einer Längsmittelebene des Gehäuses 10 miteinander verklebt oder verschweißt werden. Dieser Halbschalen können an der Berührfläche der halbierten Trennwände 12 darin eingeformt je die Halbseite einer Sammelleitung 28, 30 aufweisen.

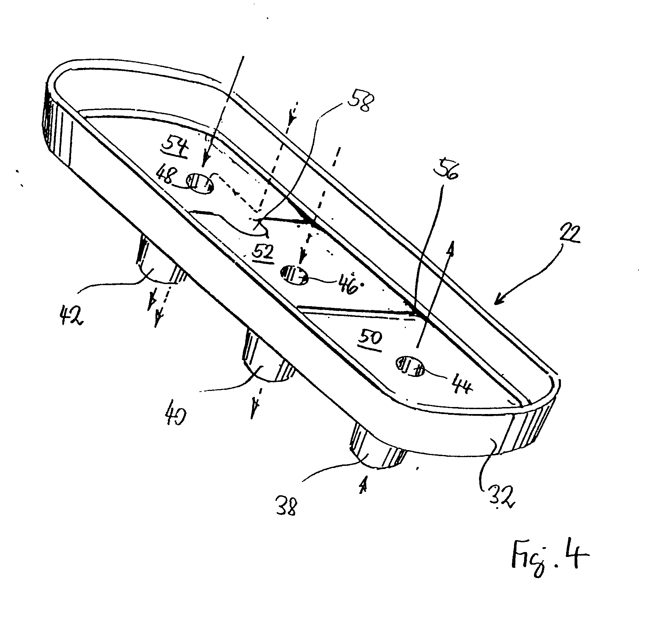

Die Deckel 20, 22 haben je eine Manschette 32, mit der sie über die Außenwand 34 des Gehäuses 10 gestülpt sind. Zwischen dem Boden 36 der Deckel 20, 22 und der Vergußmasse 26 bleibt dabei ein kleiner Zwischenraum, über den eine Strömungsverbindung zwischen den Kammern 14, 16, 18 hergestellt wird. Der untere Deckel 22 weist drei Anschlußstutzen 38, 40, 42 auf, die mit je einem Lüerverschluß versehen sind. Die Anschlußstutzen 38, 40, 42 sind mittig in einer Reihe an dem Deckel 22 angeordnet, von dessen Boden 36 sie in Gehäuselängsrichtung abstehen. Fig. 4 zeigt zugehörige Anschlußöffnungen 44, 46, 48 auf der Innenseite des Bodens 36. Die Anschlußöffnungen 44, 46, 48 liegen in Zonen 50, 52, 54 des Deckels 22, die durch eine Dichtung 56 voneinander getrennt und im aufgesetztem Zustand des Deckels 22 gegeneinander abgedichtet sind. Die Zonen sind je einer der Kammern 14, 16, 18 zugeordnet, zu deren Kapillarinnern über die Anschlußstutzen 38, 40, 42 und Zonen 50, 52, 54 eine Anschlußverbindung hergestellt wird.

Die der dritten Kammer 18 zugeordnete Zone 54 hat eine Ausbuchtung 58, die in die der zweiten Kammer 16 zugeordnete Zone 52 hineinreicht. Im Bereich dieser Ausbuchtung 58 endet die Sammelleitung 30 der zweiten Kammer 16. Es besteht somit eine Strömungsverbindung vom Ende dieser Sammelleitung 30 zu dem Anschlußstutzen 42, der an das Kapillarinnere der dritten Kammer 18 führt.

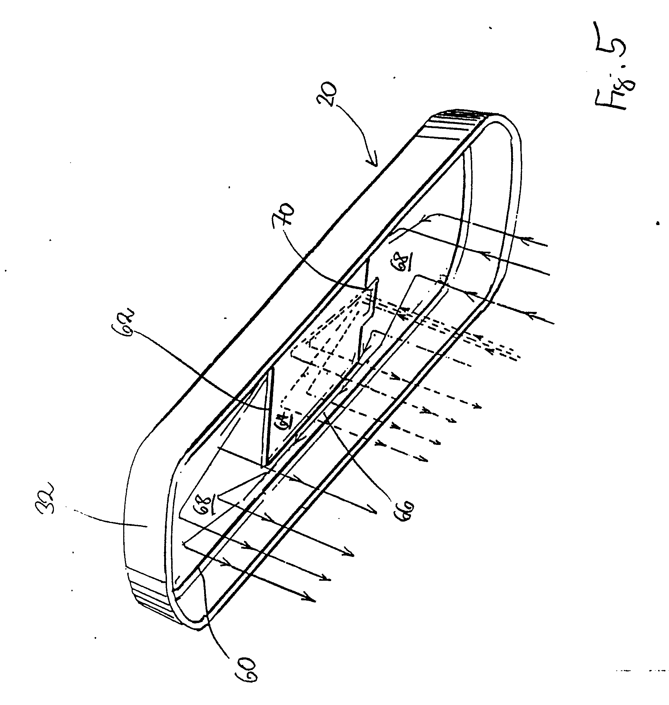

Fig. 5 zeigt den Aufbau des oberen, nicht mit Anschlußstutzen versehenen Deckels 20. Dieser weist auf der Innenseite seines Bodens 36 eine periphere Ringdichtung 60 auf. Diese umschließt eine mittig im Innern des Deckels 20 vorgesehene zweite Ringdichtung 62, die eine der zweiten Kammer 16 zugeordnete Zone 64 abteilt. An dieser vorbei führt ein zwischen den Ringdichtungen 60, 62 verbleibender Strömungsweg 66, der die oberhalb der ersten und dritten Kammer 14, 18 liegenden Deckelbereiche zu einer gemeinsamen Zone 68 verbindet. Im aufgesetzten Zustand des oberen Deckels 20 sind die Zonen 64, 68 gegeneinander abgedichtet.

Die der zweiten Kammer 16 zugeordnete mittlere Zone 64 hat eine Ausbuchtung 70, die sich auf der Seite der ersten Kammer 14 in die gemeinsame Zone 68 hineinerstreckt. Im Bereich dieser Ausbuchtung 70 endet die Sammelleitung 28 der ersten Kammer. Über die mittlere Zone 64 wird somit eine Strömungsverbindung vom Ende der ersten Sammelleitung 28 zum Kapillarinnern der zweiten Kammer 16 hergestellt. Die gemeimsame Zone 68 hingegen verbindet das Kapillarinnere der ersten und dritten Kammer 14, 18.

Die zum Abteilen der Zonen 50, 52, 54, 64, 68 verwendeten Dichtungen 56, 60, 62 sitzen vorzugsweise in entsprechenden Nuten der Deckel 20, 22 ein. Insbesondere kommt eine Verwendung von entsprechend konturierten O-Ringdichtungen in Betracht.

An der Außenwand 34 des Gehäuses 10 sind an dessen oberem und unterem Ende Anschlußstutzen 72, 74 vorgesehen, die in die dritte Kammer 18 führen und eine Anschlußverbindung zum Kapillaräußern dieser Kammer 18 herstellen. Die Anschlußstutzen 72, 74 sind für eine Hansenkupplung ausgelegt, wobei aber auch andere Formen der Anschlußverbindung möglich sind. Die Anschlußstutzen dienen zum Anschluß eines Dialysatkreises.

Im Betrieb wird das zu reinigende Blut in einem extrakorporalen Blutkreislauf mittels einer Blutpumpe an dem Anschlußstutzen 38 in die Vorrichtung eingespeist. Über die Zone 50 erfolgt eine Verteilung auf die Membrankapillaren 24 der ersten Kammer 14, deren Kapillarinneres das Blut aufsteigend durchströmt. Es findet dabei eine Filtration statt, und zwar mit Membrankapillaren 24, die für Mittelmoleküle durchlässig sind. Das Filtrat am Kapilläußern wird in der ersten Kammer 14 aufgefangen. Es tritt in die Sammelleitung 28 ein und steigt darin dank des hydrostatischen Drucks, der sich durch die Membrankapillaren 24 fortpflanzt, bis in die mittlere Zone 64 des oberen Deckels 20 auf. Dort wird das Filtrat auf die Membrankapillaren der zweiten Kammer 16 verteilt, deren Kapillarinneres es absteigend durchströmt. In der zweiten Kammer 16 findet eine Filtration des Filtrats statt, und zwar mit Membrankapillaren 24, die nur für kleinere Moleküle durchlässig sind. Das Permeat der zweiten Filtration, das am Kapillaräußern in der zweiten Kammer gesammelt wird, stellt daher eine gereinigte Flüssigkeit mit einem hohen Gehalt an Elektrolyten dar. Das Retenat der zweiten Filtration ist hingegen mit harnpflichtigen Substanzen angereichert. Es wird als Abfluß an dem Anschlußstutzen 40 abgezogen, wobei die entsprechende Strömung gedrosselt ist, beispielsweise mit einer im Schubbetrieb laufenden Pumpe.

Das in der ersten Kammer 14 gefilterte Blut gelangt über die gemeinsame Zone 68 des oberen Deckels 20 an das Kapillarinnere der dritten Kammer 18, die ein Dialysekompartiment darstellt. Das Blut durchströmt die Kapillaren der dritten Kammer 18 absteigend, wobei es dialysiert wird. Das dialysierte Blut gelangt über die abgedichtete Zone 54 des unteren Deckels 22 an den Anschlußstutzen 42, von dem aus es über den extrakorporalen Blutkreislauf an den Patienten zurückgeführt wird. Das Permeat der zweiten Filtration wird in der Sammelleitung 30 der zweiten Kammer 16 gesammelt. Wie schon erwähnt, mündet diese Sammelleitung 30 ebenfalls in die der dritten Kammer 18 zugeordnete Zone 54 des unteren Deckels 22, so daß an dieser Stelle das Permeat der zweiten Filtration mit dem dialysierten Blut vereinigt wird. Eine Zugabe von Substituat entfällt.

Die erfindungsgemäße Vorrichtung ahmt weitgehend die Funktion einer natürlichen Niere nach. Die in der ersten Kammer vorgenommene Filtration führt zur Abscheidung eines dem Primärurin vergleichbaren Filtrats. Die Filtrat-Filtration in der zweiten Kammer reichert das Filtrat zu einem Ausfluß an, der dem natürlichen Urin vergleichbar ist, und auch in ähnlicher Menge anfällt. Dabei werden Elektrolyte und Flüssigkeit zurückgewonnen. Die Dialyse in der dritten Kammer komplettiert die Blutreinigung, und die wirkt hinsichtlich des Elektrolythaushalts und der Flüssigkeitsbilanz in dem erforderlichen Maß kompensatorisch. Im Ergebnis erreicht die erfindungsgemäße Vorrichtung eine Clearance, die der bekannter Einrichtungen zur Hämodiafiltration vergleichbar oder überlegen ist, wobei aber die Zugabe von Substitutionsflüssigkeit und der damit verbundene Aufwand entfällt.

Die erfindungsgemäße Dreikammer-Blutreinigungsvorrichtung hat Einsatzmöglichkeiten über das beschriebene Verfahren hinaus. Es ist möglich, durch die Verwendung geringfügig modifizierter Deckel auch andere Verbindungen, bzw. gar keine Verbindung zwischen den einzelnen Membrankammern 14, 16, 18 herzustellen. Sieht man beispielsweise Deckel mit Anschlußstutzen sowohl am oberen als auch am unteren Ende des Gehäuses vor, so kann durch Öffnen bzw. Verschließen entsprechender Anschlüsse mit der Vorrichtung auch eine reine Hämofiltration bzw. eine reine Dialyse durchgeführt werden. Bei Verwendung entsprechender, für sehr große Moleküle durchlässige Membranen ist in der er sten Kammer 14 auch eine Plasmaseperation, und unter Einsatz geeigneter Sorbentien eine Hämoperfusion mögich.

Liste der Bezugszeichen

10 Gehause
12 Trennwand
14 Kammer 1
16 Kammer 2
18 Kammer 3
20 oberer Deckel
22 unterer Deckel
24 Membrankapillare
26 Vergußmasse
28 Sammelleitung (Kammer 1)
30 Sammelleitung (Kammer 2)
32 Manschette
34 Außenwand
36 Boden
38 Anschlußstutzen (Kammer 1)
40 Anschlußstutzen (Kammer 2)
42 Anschlußstutzen (Kammer 3)
44 Anschlußöffnung (Kammer 1)
46 Anschlußöffnung (Kammer 2)
48 Anschlußöffnung (Kammer 3)
50 Zone (Kammer 1)
52 Zone (Kammer 2)
54 Zone (Kammer 3)
56 Dichtung
58 Ausbuchtung vom 54
60 periphere Dichtung
62 innere Dichtung
64 mittlere Zone (Kammer 2)
66 Strömungsweg
68 gemeinsame Zone (Kammer 1 u.
70 Ausbuchtung von 64
72 Anschlußstutzen (Dialysat)
74 Anschlußstutzen (Dialysat)

**Ansprüche**

1. Vorrichtung zur Blutreinigung mit einem ersten Membranmodul zur Filtration des dem Patienten abgenommenen bluts unter Einsatz eines für Mittelmoleküle durchlässigen ersten Membransystems, mit einem zweiten Membranmodul zur Filtration des aus dem ersten Membranmodul kommenden Filtrats unter Einsatz eines für kleinere Moleküle durchlässigen zweiten Membransystems, wobei ein Auslaß (40) für das Retenat vorhanden ist, und mit einem dritten Membranmodul zur Dialyse des vom ersten Membranmodul kommenden gefilterten Bluts, wobei ein Auslaß für das dialysierte Blut und ein Auslaß für das Permeat des zweiten Membranmoduls verbunden und an eine zum Patienten führende Reinfusionsleitung angeschlossen sind.

2. Vorrichtung nach Anspruch 1, dadurch gekennzeichnet, daß das Membransystem des ersten Membranmoduls eine Durchlässigkeit für Moleküle mit einem Molekulargewicht bis größenordnungsmäßig ca. 50.000 Atommasseneinheiten, vorzugsweise bis größenordnungsmäßig ca. 30.000 Atommasseneinheiten, und die Membransysteme des zweiten und dritten Membranmoduls eine Durchlässigkeit für Moleküle bis größenordnungsmäßig ca. 1.800 Atommasseneinheiten haben.

3. Vorrichtung nach Anspruch 1 oder 2, dadurch gekennzeichnet, daß die wirksame Membranfläche des ersten Membransystems größenordnungsmäßig ca. 0,4 bis 0,5 m², die des zweiten Membransystems größenordnungsmäßig ca. 0,4 bis 0,6 m², und die des dritten Membransystems größenordnungsmäßig ca. 0,9 m² beträgt.

4. Vorrichtung nach einem der Ansprüche 1 bis 3, dadurch gekennzeichnet, daß sie drei in einem gemeinsamen Gehäuse (10) untergebrachte Hohlfasermembranmodule enthält.

5. Vorrichtung nach einem der Ansprüche 1 bis 4, dadurch gekennzeichnet, daß das Gehäuse (10) prismatisch oder zylindrisch und an beiden Enden mit Deckeln (20, 22) versehen ist, die eine selektive Strömungsverbindung zwischen den Membranmodulen herstellen.

6. Vorrichtung nach einem der Ansprüche 1 bis 5, dadurch gekennzeichnet, daß das Gehäuse (10) drei beidendig vergossene, je ein Bündel Hohlfasern enthaltende Kammern (14, 16, 18) aufweist, wobei die Hohlfasern mit einem an der äußeren Stirnseite offenen Ende in die Vergußmasse (26) eingebettet sind, und daß an den Dekkeln (20, 22) in deren aufgesetztem Zustand gegeneinander abgedichtete Zonen (50, 52, 54, 64, 68) abgeteilt sind, über die eine Strömungsverbindung mit dem Hohlfaserinnern der einzelnen Membranmodule besteht.

7. Vorrichtung nach einem der Ansprüch 1 bis 6, dadurch gekennzeichnet, daß die Kammern (14, 16, 18) nebeneinander liegen und druch eine gemeinsame Außenwand (34) und zwei mittlere Trennwände (12) begrenzt sind.

8. Vorrichtung nach einem der Ansprüche 1 bis 7, dadurch gekennzeichnet, daß die Kammern (14, 16, 18) kreiszylindrisch sind.

9. Vorrichtung nach einem der Ansprüche 1 bis 8, dadurch gekennzeichnet, daß an der Außenwand (34) zwei an das Hohlfaseräußere der zu dem dritten Membranmodul gehörigen Kammer (18) führende Anschlüsse (72, 74) für die Dialysatströmung vorgesehen sind.

10. Vorrichtung nach einem der Ansprüche 1 bis 9, dadurch gekennzeichnet, daß das Gehäuse (10) einstückig gefertigt, insbesondere im Spritzgußverfahren aus Kunststoff gefertigt ist.

11. Vorrichtung nach einem der Ansprüche 1 bis 9, dadurch gekennzeichnet, daß das Gehäuse (10) aus zwei Halbschalen besteht, die miteinander verklebt oder verschweißt sein können.

12. Vorrichtung nach einem der Ansprüche 1 bis 11, dadurch gekennzeichnet, daß an einem ersten (unteren) Deckel (22) drei je einem der Membranmodule zugeordnete Zonen (50, 52, 54) vorgesehen sind, die mit je einem Anschluß insbesondere in Form eines Anschlußstutzens (38, 40, 42) kommunizieren, und daß vom Hohlfaseräußern der zu dem zweiten Membranmodul gehörigen Kammer (16) ein Kanal an die dem dritten Membranmodul zugeordnete Zone (54) des unteren Deckels (22) führt.

13. Vorrichtung nach einem der Ansprüche 1 bis 12, dadurch gekennzeichnet, daß an einem zweiten (oberen) Deckel (20) eine dem ersten und dritten Membranmodul zugeordnete gemeinsame Zone (68) und innerhalb dieser eine dem zweiten Membranmodul zugeordnete Zone (64) vorgesehen sind, und daß vom Hohlfaseräußern der zu dem ersten Membranmodul gehörigen Kammer (14) ein Kanal an die dem zweiten Membranmodul zugeordnete Zone (64) des oberen Deckels (20) führt.

14. Vorrichtung nach einem der Ansprüche 1 bis 13, dadurch gekennzeichnet, daß die Kanäle mit Sammelleitungen (28, 30) verbunden sind, die sich in Gehäuselängsrichtung erstrecken.

15. Vorrichtung nach einem der Ansprüche 1 bis 14, dadurch gekennzeichnet, daß die Sammelleitungen (28, 30) im Bereich der Trennwände (12) einstückig mit dem Gehäuse (10) ausgebildet sind, wozu insbesondere Halbseiten der Sammelleitungen (28, 30) an den Halbschalen des Gehäuses (10) ausgeformt sein können.

16. Vorrichtung nach einem der Ansprüche 1 bis 14, dadurch gekennzeichnet, daß die Sammelleitungen (28, 30) von einem in die jeweilige Kammer eingelegten, endseitig offenen Rohr oder Schlauch gebildet sind.

17. Vorrichtung nach einem der Ansprüche 1 bis 16, dadurch gekennzeichnet, daß die Zonen (54, 68) im Mündungs bereich der Kanäle Einbuchtungen oder Ausbuchtungen (58, 70) haben.

18. Vorrichtung nach einem der Ansprüche 1 bis 17, dadurch gekennzeichnet, daß die Zonen (50, 52, 54, 64, 68) durch in Nuten des Deckels (20, 22) einsitzende Ringdichtungen, insbesondere O-Ringdichtungen, abgeteilt sind.

19. Verfahren zur Blutreinigung, bei dem man eine Filtration des dem Patienten abgenommenen Bluts mit einem für Mittelmoleküle durchlässigen ersten Membransystem durchführt, das gewonnene Filtrat einer zweiten Filtration mit einem nur für kleinere Moleküle durchlässigen zweiten Membransystem unterwirft, das Retenat abführt, das vom ersten Membransystem überstellte gefilterte Blut dialysiert und das dialysierte Blut mit dem Permeat des zweiten Membransystems vereinigt und ohne Zugabe vom Substituat zum Patienten zurückführt.

Fig. 1

Fig. 2

Fig. 3

0 264 695

Fig. 4

Fig.5

0 264 695